# EUROPEAN PATENT APPLICATION

(11) **EP 4 717 699 A1**
(43) Date of publication of application: **01.04.2026**
(21) Application number: 24811393.8
(22) Date of filing: 22.05.2024
(51) Int. Cl.: C07K 1/14, C07K 1/16, A61K 8/98, A61K 8/64, A61Q 19/00, A61Q 19/08, A61K 35/16, A61K 38/18, A61P 17/02

(54) **METHOD FOR CONCENTRATING REGENERATION FACTOR IN BLOOD BY USING AQUEOUS TWO-PHASE SYSTEM**

(30) Priority: 23.05.2023 KR 20230065996
(71) Applicant: Pnaseer Inc., Suwon-si Gyeonggi-do 16514 (KR)
(72) Inventor: SHIN, Hyunwoo, Gyeonggi-do 16508 (KR)
(74) Representative: Müller, Christian Stefan Gerd
(86) International application number: PCT/KR2024/006894
(87) International publication number: WO 2024/242454

(57) **Abstract**

More specifically, the method enables the efficient extraction and concentration of regeneration factor-rich plasma (RRP) with regenerative efficacy in a short time, and the RRP manufactured using this method promotes cell proliferation, making it applicable for regenerative therapy and skin beauty.

## Description

### Technical Field

This invention relates to customized healthcare and innovative new drugs, specifically to a method for extracting and concentrating regenerative factors from blood using an aqueous two-phase system to produce regenerative factor-rich plasma (RRP), and a method for applying this to cosmetic and wound treatment.

### Background Art

Regenerative proteins (regenerative factors) present in blood are also known as growth factors and are proteins involved in cell growth, proliferation, and differentiation, including bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

Aqueous two-phase system is a method of concentrating and extracting particles by creating two aqueous phases. This involves two polymers, one polymer and one kosmotropic salt, or two salts (one chaotropic salt and the other a kosmotropic salt) are mixed at appropriate concentrations or specific temperatures. Aqueous phase separation is primarily composed of water and non-volatile components, effectively removing volatile organic compounds. It has been used for years as a non-denaturing and positive separation medium in biotechnology applications. However, the types and concentrations of substances required to form an aqueous two-phase system is critical for effectively concentrating and extracting target materials, and to date, no method has been developed to concentrate and extract regenerative factor-rich plasma using an aqueous two-phase system. Nevertheless, the present invention has developed a method for manufacturing regenerative factor-rich plasma containing more than five times the amount of regeneration factors and proteins compared to whole blood using the aqueous two-phase system, thereby completing the invention despite the aforementioned difficulties.

### Technical Problem

One objective of the present invention is to provide a method for extracting or concentrating regeneration factor-rich plasma (RRP) from blood using an aqueous two-phase system.

Another objective of the present invention is to provide a skin beauty method, a skin beauty cosmetic, or a skin beauty composition containing RRP extracted or concentrated by the aforementioned method.

Another objective of the present invention is to provide a wound treatment composition or a bone disease treatment composition containing RRP extracted or concentrated by the aforementioned method.

Another objective of the present invention is to provide a device for extracting or concentrating RRP (Regeneration Factor-Rich Plasma) from blood using an aqueous two-phase system, comprising: a rubber stopper (10); a lid (20) located below the rubber stopper; a main body (30) connected to the lid and performing centrifugation to separate RRP; a rubber stopper (40) located at the lower portion of the main body (30); and a support (50) connected to the main body (30) and the rubber stopper (40) and located at the lower portion of the main body, thereby providing a device for extracting or concentrating RRP (Regeneration Factor-Rich Plasma) from blood using an aqueous two-phase system.

However, the technical problem to be achieved by the present invention is not limited to the above-mentioned problems, and other problems that are not mentioned will be clearly understood by a skilled person in the art from the following description.

### Solution to Problem

Various embodiments of the present invention are described with reference to the accompanying drawings. In the following description, various specific details, such as specific forms, compositions, and processes, are described for a complete understanding of the present invention. However, specific embodiments may be implemented without one or more of these specific details or in combination with other known methods and forms. In other examples, known processes and manufacturing techniques are not described in detail to avoid making the present invention unnecessary or ambiguous. A reference throughout this specification to a single embodiment means that the special features, forms, compositions, or characteristics described in connection with that embodiment are included in one or more embodiments of the present invention. Therefore, the situations of embodiments described at various locations throughout this specification do not necessarily represent the same embodiments of the invention. Additionally, special features, forms, compositions, or characteristics may be combined in any suitable manner in one or more embodiments.

To achieve the above objectives,
the present invention comprises: (a) a step of extracting plasma from extracted whole blood; (b) a step of removing platelets and exosomes from the plasma; (c) a step of extracting and concentrating regeneration factor-rich plasma from the plasma using an aqueous two-phase system; (d) purifying impurities from regeneration factor-rich plasma); and (e) selectively extracting regeneration factors from regeneration factor-rich plasma), thereby providing a method for manufacturing regeneration factor-rich plasma material.

In another embodiment of the present invention, the concentration step (b) is performed using centrifugation or chromatography, wherein platelets are removed by centrifugation at 100g force for 10 minutes, followed by ultracentrifugation at 10,000g force for 2 hours, and exosome removal is achieved using agarose gel chromatography.

In another embodiment of the present invention, the concentration step (c) involves extracting and concentrating RRP (Regeneration Factor-Rich Plasma) using 4.3% sodium sulfate, 16% PEG (molecular weight 8,000), and 3% hyaluronic acid. In another embodiment of the present invention, the concentration of the sodium sulfate may be 1.0 to 10.0 wt%, 1.2 to 5.3 wt%, 2.5 to 5.3 wt%, 3.0 to 5.0 wt%, 3.5 to 5.0 wt%, 3.0 to 9.0 wt%, 3.0 to 8.5 wt%, 3.0 to 8.0 wt%, or 3.5 to 8.0 wt%.

In other embodiments of the present invention, the concentration of the PEG may be 5 to 20 wt%, 7 to 20 wt%, 8 to 20 wt%, 9 to 20 wt%, 10 to 20 wt%, or 10 to 18 wt%.

In other embodiments of the present invention, the hyaluronic acid may be 1 to 10 wt%, 1 to 8 wt%, 1 to 7 wt%, 1 to 6 wt%, 2 to 6 wt%, 2 to 7 wt%, or 1 to 9 wt%.

In other embodiments of the present invention, the average molecular weight of hyaluronic acid may be 100,000 or more and 500,000 or less.

In some cases, hyaluronic acid can be replaced with other additives, and applicable additives include (NH₄)₂SO₄, Na₂SO₄, MgSO₄, K₂HPO₄, KH₂PO₄, NaCl, KCl, NaBr, NaI, LiCl, n-Bu₄NBr, n-Pr₄NBr, Et₄NBr, Mg(OH)₂, Ca(OH)₂, Na₂CO₃, ZnCO₃, Ca₃(PO₄)₂, ZnCl₂, (C₂H₃)₂Zn, ZnCO₃, CdCl₂, HgCl₂, CoCl₂, (CaNO₃)₂, BaCl₂, MgCl₂, PbCl₂, AlCl₃, FeCl₂, FeCl₃, NiCl₂, AgCl, AuCl₃, CuCl₂, sodium dodecyl sulfate, sodium tetradecyl sulfate, dodecyltrimethylammonium bromide, dodecyltrmethylammonium chloride, and tetradecyltrimethylammonium bromide are available.

Additionally, available additives may be one hydrophilic polymer selected from the group consisting of polyarginine, polylysine, polyethylene glycol, polypropylene glycol, polyethylene imine, chitosan, protamine, polyvinyl acetate, hyaluronic acid, chondroitin sulfate, heparin, alginate, hydroxypropyl methylcellulose, gelatin, starch, poly(vinyl methyl ether ether), polyvinylpyrrolidone, and a combination thereof.

Additionally, it may be one hydrophilic polymer selected from the group consisting of cyclodextrin, glucose, dextran, mannose, sucrose, trehalose, maltose, ficoll, inositol, mannitol, sorbitol, sucrose-mannitol, glucose-mannitol, trehalose-polyethylene glycol, sucrose-polyethylene glycol, sucrose-dextran, and combinations thereof.

In another embodiment of the present invention, the purification step (d) removes HDL or LDL using a chromatography filter immobilized with Apo A and Apo B antibodies, wherein the pore size of the filter is preferably 1 µm or more and 10 µm or less.

In another embodiment of the present invention, the selective extraction step (e) preferably comprises attaching the selected protein to a chromatography filter on which an anti-regenerative factor antibody is immobilized, and then recovering the selected protein from the filter using a preservation buffer. Available antibodies include, but are not limited to, antibodies against bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

Additionally, the present invention includes a device capable of performing the method of the present invention, and (g) provides a step for extracting and concentrating RRP from plasma.

(g) the device capable of performing the step of extracting and concentrating RRP from plasma can be implemented in two types. The first type is manufactured using an automated pipette, and the second type is implemented in a centrifugation container that can easily extract the concentrated RRP with a syringe after centrifuging whole blood.

The present invention will now be described in detail.

While there have been cases of therapeutic applications utilizing platelet concentrates in plasma, the heterogeneous nature of platelets in blood has made approval for these treatments challenging. To date, these treatments have only been approved for use in medical procedures. Stem cell therapies and stem cell-derived exosome therapies also face challenges in gaining approval as therapeutic agents due to their heterogeneous nature.

This application proposes a method and device for extracting bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF) with high efficiency and purity by concentrating RRP in blood. There have been no cases of therapeutic materials being developed using this method.

In addition, conditions or procedures to which existing RRP can be applied include Temporomandibular Disorders, Gastric Ulcer Caused by Endoscopic Submucosal Dissection, Human Autologous Fat Transfer, the Treatment of Knee Osteoarthritis, the Treatment of Chronic Non-Healing Ulcers, Hair Restoration Therapy for Androgenetic Alopecia, Treatment for ACHILLES Tendon Tears, High Risk Proliferative Diabetic Retinopathy, High Risk Proliferative Diabetic Retinopathy, Knee OA, Patella-Dislocation, Lumbar Facet Joint Syndrome, Retinitis Pigmentosa, Diabetic Macular Edema, Traumatic Bone Cyst, High Risk Proliferative Diabetic Retinopathy, Scar, Dry Eye, Sjogren Syndrome, Osteoarthritis, Meniscus Lesion, Melasma, Muscle Injury, Ulnar Neuropathy at Elbow, Chronic Ulcer, Alopecia, Epicondylitis, Bone Resorption, Rotator Cuff Tears, Thin Endometrium, Striae Gravidarum, Plantar Fascitis, Coxarthrosis, Osteoarthritis, Muscle Tear, Tennis Elbow, Skin Graft, Aging, Osteo Arthritis Knee.

### 1. Method for extracting or concentrating regeneration factor-rich plasma (RRP)

In one embodiment of the present invention, a method for extracting or concentrating regeneration factor-rich plasma (RRP) from blood using an aqueous two-phase system is provided.

In the present invention, the term "aqueous two-phase system (ATPS)" refers to a system formed by two immiscible aqueous solutions that can separate liquid-liquid and effectively extract, separate, purify, and concentrate proteins, membranes, viruses, enzymes, nucleic acids, and other biomolecules. However, recent studies on the aqueous two-phase system have shown relatively promising results with high yields; however, due to the nonspecific separation mechanisms used in the aqueous two-phase system process, only modest purification factors have been obtained in some cases.

The key process parameters associated with an aqueous two-phase system are all related to the partitioning behavior of the process stream components. For example, whether molecules are separated into the upper or lower phase is determined by the characteristics of the molecules (e.g., charge, MW, and solubility) and the polymer (e.g., concentration, MW, and hydrophobicity), while the physicochemical environment of the system (e.g., temperature, pH, and ionic strength) also affects the yield and purification factors of the concentration and extraction processes in aqueous phase separation. To ensure optimal performance, various interaction factors between different operating parameters and careful balancing are necessary. Additionally, an aqueous two-phase system often faces challenges due to limited basic knowledge about the separation of biological components, making it difficult to obtain the desired target material with high yield. Therefore, process optimization requires an experimental design approach based on fundamental knowledge of biological components, along with extensive screening of various conditions. As a result, the development of new aqueous two-phase system is a time-consuming and labor-intensive endeavor.

Despite these limitations, the aqueous two-phase system according to the present invention utilizes the aqueous two-phase system to create two aqueous phases and concentrate and extract particles, thereby enabling the high-yield concentration and extraction of regeneration factor-rich plasma (RRP) from blood.

In the present invention, "regeneration factor" refers to a protein that is also known as a growth factor and is involved in cell growth, proliferation, and differentiation. These regeneration factors bind to specific receptors depending on their type and exhibit their respective functions, acting as cellular signaling molecules. By using these regeneration factors as delivery vehicles in tissue engineering alongside biomaterials, cell growth and differentiation can be promoted, thereby achieving effective tissue regeneration. Additionally, the introduction of an appropriate drug delivery system for growth factors, which are crucial for cell growth and differentiation, is also highly important when combined with suitable biomaterials for tissue regeneration. However, these regenerative factors face significant challenges in isolation and purification. These proteins associated with regeneration (regenerative factors) influence the three stages of wound healing-the inflammatory phase, the trophic phase, and remodeling-but they also have drawbacks such as short biological half-lives, lack of sustained stability, and limited tissue specificity.

The regenerative factors or growth factors include bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), hepatocyte growth factor (HGF), and others, though this list is not exhaustive. Among the regenerative factors, important ones include epidermal growth factor (EGF) and platelet-derived growth factor (PDGF), with platelet-derived growth factor (PDGF) being particularly significant due to its abundant presence in regenerative factor-rich plasma (RRP).

More specifically, the wound healing process involves epidermalization, wound contraction, collagen synthesis, and the formation of granulation tissue. In full-thickness skin wounds, wound contraction plays a primary role, while in partial-thickness skin wounds where skin appendages remain, healing through epidermalization is more important than contraction. During wound healing, various factors promote the migration, division, and proliferation of wound healing cells to the wound site. These factors include chemotactic agents (chemo attractants, attachment factors) and growth-promoting factors, among others.

Among the various growth factors known to date, the most effective and clinically applicable ones for wound healing are epidermal growth factor (EGF) and platelet-derived growth factor (PDGF). EGF, first discovered in 1961, has been shown through *in vitro* and animal experiments to act as a strong mitogen on epidermal cells, significantly influencing epidermalization. PDGF was discovered in 1985 and is known to induce chemotactic activity and mitogenic activity in fibroblasts, promoting collagen deposition and granulation tissue formation at wound sites. It is reported to have a greater influence on the proliferative and contraction phases of wound healing rather than the epidermalization stage.

In this invention, the term "epidermal growth factor (EGF)" refers to a protein that binds to the receptor EGFR to stimulate cell growth and differentiation. Human EGF has a molecular weight of 6 kDa, consists of 53 amino acid residues, and contains three intramolecular disulfide bonds. Additionally, among regenerative factors, epidermal growth factor is known to play a key role in skin regeneration and anti-aging, making it a functional cosmetic ingredient. Furthermore, epidermal growth factor is not only listed in the International Cosmetic Ingredient Dictionary (ICID) of the Cosmetic Ingredient Review (CIR) in the United States but also approved as a cosmetic ingredient by the Ministry of Food and Drug Safety (MFDS) in Korea, enabling its official use as a cosmetic ingredient both domestically and internationally (MFDS Notice No. 2006-12, April 12, 2006).

In this invention, the "platelet-derived growth factor (PDGF)" is one of numerous growth factors that regulate cell growth and division. In particular, PDGF plays a crucial role in the mitosis, i.e., proliferation, of mesenchymal cells such as mesenchymal stem cells, vascular smooth muscle cells, and osteoblasts, as well as in the differentiation of mesenchymal cells into various types of cells, thereby contributing to tissue regeneration. bone cells, vascular smooth muscle cells, and mesenchymal stem cells, thereby playing a crucial role in their proliferation. As a result, PDGF is used in medical fields to aid in the treatment of chronic ulcers and as an alternative to bone autografts to stimulate bone regeneration and repair in orthopedic surgery and periodontal treatment. Additionally, PDGF is a protein necessary for the cell division of fibroblasts, a type of connective tissue cell, during wound healing. Specifically, PDGF administered to mononuclear-macrophage cells and fibroblasts stimulates chemotaxis, proliferation, and gene expression, significantly increasing the influx of inflammatory cells and fibroblasts, thereby accelerating the formation of the extracellular matrix and collagen and shortening the healing process.

In this invention, "regeneration factor-rich plasma (RRP)" refers to plasma enriched with proteins related to regeneration that are present in blood, and does not include platelets or exosomes. However, RRP (Regeneration factor-rich Plasma) may include platelets or exosomes containing regenerative factors depending on the intended use. The RRP (Regeneration factor-rich Plasma) extracted and concentrated by the method of the present invention contains 3-5 times more RRP (Regeneration factor-rich Plasma) than whole blood when compared to whole blood. RRP promotes regeneration or enables proliferative therapy through the roles related to regeneration described above at concentrations higher than physiological levels. RRP (Regeneration factor-rich Plasma) can assist in the treatment and regeneration of various tissues, including bone, cartilage, tendons, ligaments, and muscles, as well as in the fields of maxillofacial and plastic surgery.

### 2. Skin beautification method, cosmetic composition, and cosmetic formulation using extracted or concentrated RRP (Regeneration factor-rich Plasma)

In other embodiments of the present invention, skin care methods, skin care cosmetics, and skin care compositions comprising RRP (Regeneration factor-rich Plasma) extracted or concentrated by a method according to any one of claims 1 to 12 are provided.

In the present invention, RRP (Regeneration factor-rich Plasma) containing five times or more regeneration factors and proteins compared to whole blood is manufactured using aqueous two-phase system, and further concentrated regeneration factor proteins, bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), hepatocyte growth factor (HGF), etc., by chromatography, and applying them to cosmetic and therapeutic uses.

In a preferred embodiment of the present invention, the skin care composition or cosmetic composition is preferably a cosmetic for preventing skin aging, but is not limited thereto. In the present invention, anti-aging may mean preventing, delaying, and/or improving aging phenomena caused by internal factors, including genetic factors, and external factors, including ultraviolet rays. For example, in the present invention, anti-aging refers to the efficacy of slowing down, blocking, or preventing the aging process known in the art, which may include inhibiting bodily aging or enhancing bodily vitality. Specifically, in the present invention, anti-aging may involve inhibiting cellular aging related to cellular metabolic processes, division, or immune responses.

In particular, the anti-aging cosmetic composition provided in the present invention comprises a rich amount of regeneration factor-rich plasma (Regeneration factor-rich Plasma), and may prevent, delay, and/or improve skin aging phenomena caused by internal factors, including genetic factors, and external factors, including ultraviolet rays.

The cosmetic composition of the present invention can be used in various cosmetic products such as cosmetic water, nutrient lotion, nutrient essence, massage cream, beauty bath additive, body lotion, body milk, bath oil, baby oil, baby powder, shower gel, shower cream, sunscreen lotion, sunscreen cream, sun tan cream, skin lotion, skin cream, UV-blocking cosmetics, cleansing milk, hair loss agent (cosmetic), face and body lotion, face and body cream, skin whitening cream, hand lotion, hair lotion, cosmetic cream, jasmine oil, bath soap, liquid soap, cosmetic soap, shampoo, hand cleanser (hand cleaner), medicinal soap {non-medical}, cream soap, facial wash, body cleanser, scalp cleanser, hair rinse, cosmetic soap, tooth whitening gel, toothpaste, etc. For this purpose, the composition of the present invention may further include solvents commonly used in the manufacture of cosmetic compositions, or appropriate carriers, excipients, or diluents.

The types of solvents that may be further added to the cosmetic composition of the present invention are not particularly limited, but examples include water, saline solution, DMSO, or combinations thereof. Carriers, excipients, or diluents may include purified water, oils, waxes, fatty acids, fatty alcohols, fatty acid esters, surfactants, humectants, thickeners, antioxidants, viscosity stabilizers, chelating agents, buffers, and low-grade alcohols, but are not limited to these. Additionally, depending on the application, whitening agents, moisturizers, vitamins, UV blockers, fragrances, dyes, antibiotics, antibacterial agents, and antifungal agents may be included.

The oils used may include hydrogenated vegetable oils, castor oil, cottonseed oil, olive oil, palm oil, jojoba oil, and avocado oil. Waxes may include beeswax, carnauba wax, candelilla wax, montan wax, ceresin, liquid paraffin, and lanolin.

Fatty acids include stearic acid, linoleic acid, linoleic acid, and oleic acid can be used as fatty acids. Fatty alcohols include cetyl alcohol, octyl dodecanol, oleyl alcohol, panthenol, lanolin alcohol, stearyl alcohol, and hexadecanol, while fatty acid esters include isopropyl myristate, isopropyl palmitate, and butyl stearate. Surfactants include cationic surfactants, anionic surfactants, and nonionic surfactants known in the cosmetics industry, and surfactants derived from natural sources are preferred whenever possible.

Additionally, the composition may include humectants, thickeners, antioxidants, and other ingredients commonly used in the cosmetics industry, with their types and quantities following industry standards.

### 3. Wound treatment composition and regenerative composition using extracted or concentrated RRP (Regeneration factor-rich Plasma)

In another embodiment of the present invention, a wound treatment composition and a regenerative composition containing extracted or concentrated RRP (Regeneration factor-rich Plasma) are provided.

In the present invention, the term "wound" is preferably selected from the group consisting of wounds, bedsores, burns, abrasions, puncture ulcer wounds, stab wounds, chronic skin wounds caused by active oxygen, bruises, cuts, wounds of the mucous membrane of the throat or mouth, lacerations, diabetic ulcers, leg ulcers, hypertensive ischemic ulcers, venous ulcers, and foot ulcers, but is not limited thereto.

Additionally, the pharmaceutical composition of the present invention may further include appropriate carriers, excipients, or diluents in accordance with conventional methods. Examples of carriers, excipients, and diluents that may be included in the pharmaceutical composition of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, methyl hydroxybenzoate, propyl hydroxybenzoate, talc, magnesium stearate, and mineral oil, among others, but are not limited to these.

Furthermore, the pharmaceutical composition according to the present invention may be formulated into oral dosage forms such as tablets, granules, capsules, suspensions, emulsions, syrups, aerosols, etc., or into topical preparations, suppositories, or sterile injectable solutions, using conventional methods.

In detail, when formulating, it can be prepared using diluents or excipients such as fillers, bulking agents, binders, wetting agents, disintegrants, and surfactants that are commonly used.

Solid dosage forms for oral administration include tablets, capsules, granules, and powders, and these solid dosage forms may be prepared by mixing the pharmaceutical composition of the present invention with at least one excipient, such as starch, calcium carbonate, sucrose, lactose, gelatin, etc., to prepare the formulation. Additionally, lubricants such as magnesium stearate and talc may be used in addition to simple excipients.

For oral liquid formulations, examples include suspensions, solutions, emulsions, and syrups. In addition to commonly used simple diluents such as water and liquid paraffin, various excipients, such as humectants, sweeteners, flavorings, and preservatives, may be included.

Formulations for non-oral administration include sterile aqueous solutions, non-aqueous solvents, suspensions, emulsions, freeze-dried formulations, and suppositories.

Non-aqueous solvents and suspensions may include propylene glycol, polyethylene glycol, plant-based oils such as olive oil, and injectable esters such as ethyl oleate. Excipients for suppositories include witepsol, macrogol, tween 61, cocoa butter, lauric acid, and glycerol gelatin.

The administration routes for the pharmaceutical compositions according to the present invention are not limited to these, but include oral, intravenous, intramuscular, intraarterial, intra-osseous, intra-meningeal, intra-cardiac, transdermal, subcutaneous, intra-abdominal, intranasal, intestinal, local, sublingual, or rectal. Oral or non-oral administration is preferred.

The term "non-oral" as used herein includes subcutaneous, intradermal, intravenous, intramuscular, intra-articular, intra-synovial, intra-sternal, intra-meningeal, intra-lesional, and intracranial injection or infusion techniques. The pharmaceutical compositions of the present invention may also be administered in the form of suppositories for rectal administration.

The pharmaceutical compositions of the present invention may vary depending on various factors, including the activity of the specific compounds used, age, weight, general health, gender, condition, administration time, administration route, excretion rate, drug combination, and the severity of the specific disease to be prevented or treated. The dosage of the pharmaceutical compositions may vary depending on the patient's condition, weight, severity of the disease, drug form, route of administration, and duration of treatment, but may be appropriately selected by those skilled in the art, and may be administered at a dosage of 0.0001 to 50 mg/kg or 0.001 to 50 mg/kg per day. Administration may be once daily or divided into multiple doses. The above dosage does not limit the scope of the present invention in any way. The pharmaceutical composition according to the present invention may be formulated as tablets, capsules, capsules, liquids, gels, syrups, slurries, or suspensions.

### 4. Containers used for extracting or concentrating RRP or RRP and platelets

In one embodiment of the present invention, a container for extracting or concentrating RRP according to the method is provided, comprising: a body (100) that provides an internal space for receiving blood and extracting or concentrating RRP during centrifugation; a first lid (200) provided on one side of the body (100) to inject the blood or a separate substance into the internal space of the body; and a second lid (300) provided on the other side of the body (100) opposite to the one side to discharge the RRP extracted or concentrated from the body (100).

In the present invention, the term "container" is not particularly limited to containers generally used for centrifugation or an aqueous two-phase system, and may preferably be a medical container. Additionally, while the present invention discloses a container for extracting or concentrating RRP, this is merely one embodiment, and the container may also be used to extract or concentrate RRP and platelets simultaneously, or to extract and concentrate both RRP and platelets for application to the skin using a syringe adapter. That is, the structure is configured such that the body (100) converges toward the lower portion, and when combined with a syringe, it can be sealed while also having an appropriately sized hole at the lower portion for easy application to the skin or other surfaces. As medical containers, examples include syringes used for the outer barrel (hereinafter referred to as the "syringe") of a syringe and for filling with a solution or medication (hereinafter also referred to as a "pre-filled syringe"), and containers used for filling with a solution or medication (hereinafter also referred to as "solution storage containers"). Here, a pre-filled syringe refers to a syringe-shaped formulation in which a pharmaceutical solution or drug is pre-filled, and it includes a single-chamber type filled with one type of liquid and a double-chamber type filled with two types of drugs. Most pre-filled syringes are single-chamber types, but for double-chamber types, there are formulations consisting of a powder and its solution (liquid-powder type) and formulations consisting of two types of liquids (liquid type). Syringes used for syringes and pre-filled syringes include, for example, pre-fillable syringes, vaccine-specific pre-filled syringes, anticancer drug-specific pre-filled syringes, and needle-free syringes. For liquid drug storage containers, examples include ampoules, vials, bottles, vials, infusion bottles, bulk containers, test tubes, and analysis cells. More specifically, containers for liquid, powder, or solid medications, such as ampoules, press-through packages, infusion bags, dropper containers, and eye drop containers; sample containers for blood testing, such as sampling test tubes, blood collection tubes, and specimen containers; analytical containers, such as ultraviolet inspection cells; sterilization containers for medical devices such as scalpels, forceps, gauze, and contact lenses; disposable syringes, pre-filled syringes, and other medical devices; laboratory equipment such as beakers, vials, ampoules, test tube flasks, and others; and housings for artificial organs, among others.

In the present invention, the "body (100)" refers to a structure that accommodates blood and provides an internal space where RRP is extracted or concentrated during centrifugation, and is not specifically limited to structures used for centrifugation or aqueous two-phase system. Additionally, while it is preferably designed to provide an internal space for extracting or concentrating RRP, it is not specifically limited to this purpose and may also be used to provide an internal space for extracting or concentrating various biological materials.

In the present invention, the "first lid (200)" is not specifically limited to a structure capable of sealing the container during RRP or RRP and platelet extraction and concentration via centrifugation or aqueous two-phase system. Preferably, it is an elastic structure capable of sealing the container, such as a rubber cap.

In another embodiment of the present invention, the first lid (200) is provided on a cover (120) disposed on the upper side of the body (100) to open and close the body (100), thereby providing a container for extracting or concentrating RRP.

Additionally, the "first lead (200)" is openable and closable and may be separated vertically, with a portion of the first lead being fixed to the fixing member (122). In this way, during the opening and closing process, the first lead (200) can be easily opened or closed by being fixed to the fixed member (122), and by fixing one side of the first lead to the fixed member, the movement of the first lead during the opening and closing process can be restricted, thereby preventing the first lead from separating and being lost. Additionally, the first lead (200) may be configured to be of a suitable size to accommodate the main body of the syringe. Preferably, it is made of an elastic material such as rubber, which can seal the main body of the syringe when it is inserted.

In the present invention, the "second lead (300)" is openable and closable and may be configured to be of a size suitable for fitting the main body of the syringe. Preferably, it is made of an elastic material such as rubber that can seal the main body of the syringe when fitted. As described above, the container used for extracting or concentrating RRP according to the above method has a first lead (200) and a second lead (300) at the top and bottom, allowing the syringe to be inserted at the top or bottom, thereby enabling the extraction of any of the materials separated vertically. Additionally, when extracting material through the syringe in this case, opening the opposite lead maintains a constant pressure inside the device, allowing the material to be easily removed with the syringe. Furthermore, when only one of the two leads is opened, the pressure balance between the internal pressure of the body (100) and the external atmospheric pressure prevents the liquid inside from flowing out. Specifically, when using the container, closing the upper lead prevents the liquid inside the body from flowing out even when the lower lead is opened.

In another embodiment of the present invention, the body (100) is formed such that the opposite side of the body (100) narrows toward the second lead (300), thereby providing a container for extracting or concentrating RRP. In this way, the opposite side of the body (100) is formed to narrow toward the second lead (300), enabling even small amounts of biological material to be withdrawn through the lower part using a syringe. When used as a syringe adapter for applying biological material to the skin or other surfaces, this design allows for precise application of even small amounts of biological material. Additionally, the body (100) may include graduations to indicate the volume.

In another embodiment of the present invention, the container for extracting or concentrating RRP further includes a support (140) that wraps around the outer surface of the body (100) on the opposite side of the second lead (300). As previously described, the opposite side of the body (100) is formed to narrow toward the second lead (300), which may have the drawback of being unstable during centrifugation, potentially breaking or requiring storage in a horizontal position. by further including the support structure (140), the body (100) is sturdily supported during centrifugation, and the container can be stored upright, thereby improving storage convenience.

Additionally, to allow the container for extracting or concentrating RRP according to the present invention to be stably mounted on the device performing centrifugal separation, mounting grooves (144) may be provided on the outer surface of the support member (140).

For extracting plasma regeneration material:
1. Open the rubber cap on the top surface and inject 20 ml of blood into the main body.
2. Close the rubber cap and perform centrifugation.
3. Open the lower rubber cap, insert a 20 ml syringe into the main body, then open the upper rubber cap and pull the syringe to remove the red blood cell and platelet layer into the syringe.
4. Close the lower rubber stopper, open the upper rubber stopper, inject the polymer, and mix thoroughly.
5. Close the upper rubber stopper and perform centrifugation.
6. The separated regenerative material at the bottom can be extracted using the same procedure as step 3.

To extract platelets and regenerative material together,
1. Open the upper rubber stopper and inject 20 ml of blood into the main body.
2. Add the polymer to the main body.
3. Close the rubber stopper and perform centrifugation.
4. Open the rubber stopper at the bottom, insert a 20 ml syringe into the main body, open the rubber stopper at the top, and pull the syringe to remove the red blood cells.
5. Insert a 5 ml syringe into the bottom and use it in the order of extracting platelets and regenerative material layers.

To use as a syringe adapter for application,
1. Prepare the sample using the method described above for extracting plasma regenerative material and extracting platelets and regenerative material together, and prepare a new, unused device.
2. Open the upper rubber stopper and inject the sample into the main body.
3. To accurately dispense the injected sample onto the skin, attach a 20ml syringe filled with air to the top, and open the support and rubber stopper.
4. Press the syringe inserted at the top to apply the medication from the main body in the downward direction.

### Advantageous Effects of Invention

This invention enables the concentration and extraction of regeneration factor-rich plasma (RRP) with a high yield from blood, and the regeneration factors in RRP, such as bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), hepatocyte growth factor (HGF), and others can be extracted and purified with high efficiency to manufacture the product.

### Brief Description of Drawings

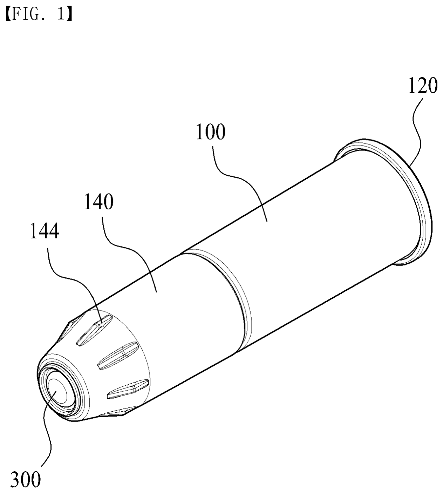

FIG. 1 is a perspective view of an embodiment of a container for extracting or concentrating regeneration factor-rich plasma (RRP) according to the present invention.

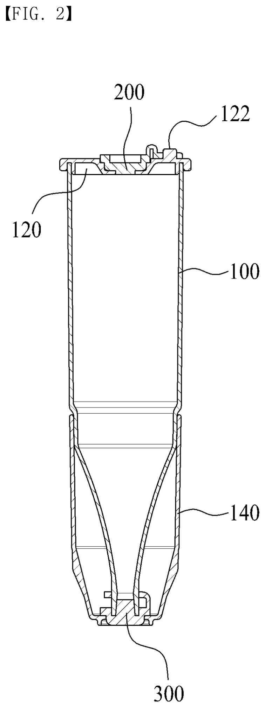

FIG. 2 is a cross-sectional view illustrating an embodiment of a container for extracting or concentrating RRP according to the present invention, as shown in Figure 1.
FIG. 3 is the results confirming that nanoparticles have been removed when following the method for extracting or concentrating RRP according to the present invention.
FIG. 4 is an ELISA result confirming that EGF was concentrated by a maximum of approximately 8.7 times compared to before concentration when following the method for extracting or concentrating RRP according to the present invention.
FIG. 5 is an ELISA results confirming that PDGF was concentrated to a maximum of approximately 10.518 times compared to before concentration when following the RRP extraction or concentration method according to the present invention.
FIG. 6 is the results of an MTT analysis demonstrating that the RRP provided by the present invention exhibits antioxidant effects on human skin cells.

### Description of Reference Numerals

100: Body
120: Cover
   122: Fixing member
140: Support
   144: Mounting slot
200: First lead
300: Second lead

### Detailed Description of Invention

Hereinafter, the present invention will be described in more detail by way of examples. These examples are only for describing the present invention in more detail, and it will be apparent to those skilled in the art that according to the gist of the present invention, the scope of the present invention is not limited by these examples.

### Example

### [Preparation Example] Removal of exosomes (exosomes)

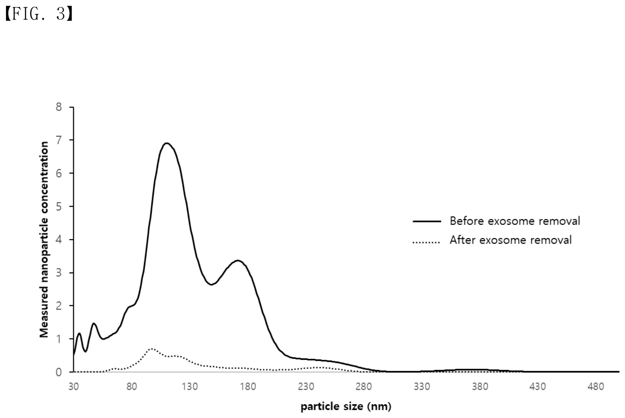

As a specific example, bovine blood was used. Purchased bovine blood was completely thawed at room temperature and then aliquoted into 50 mℓ conical tubes. The bovine blood was centrifuged at 200 g force for 10 minutes, and the supernatant was extracted using a pipette. The extracted supernatant was centrifuged at 3000 g for 30 minutes, and the supernatant was extracted again. The extracted supernatant was centrifuged at 100,000 g for 2 hours, and the supernatant was extracted. The extracted supernatant was centrifuged at 100,000 g for 2 hours, and the supernatant was extracted. Using nano particle tracking analysis, it was confirmed that 92.3%±14 of nano particles with a size of 30 nm or larger, including exosomes, were removed, as shown in Figure 3.

### [Example 11 Formation of an aqueous two-phase system

Exosomes were removed from the sample, and salts and polymers were added to achieve the concentrations listed in Table 1. The sample was mixed by vortexing and incubated at room temperature for 30 minutes. The sample was centrifuged at 1000 g for 30 minutes, and the concentrated RRP was extracted from the lower layer. At this point, systems A, B, and C in Table 1 formed an aqueous two-phase system, while systems D and E did not form an aqueous two-phase system.

**[Table 1]**

| | Sodium sulfate (%) | PEG (%) | Hyaluronic acid (%) |
|---|---|---|---|
| System A | 4.3 | 16 | 3 |
| System B | 2.8 | 14 | 5 |
| System C | 7 | 12 | 3.5 |
| System D | 2.5 | 5 | 5 |
| System E | 1 | 3 | 12 |

### [Example 2] Regeneration factor-rich plasma (RRP) concentration

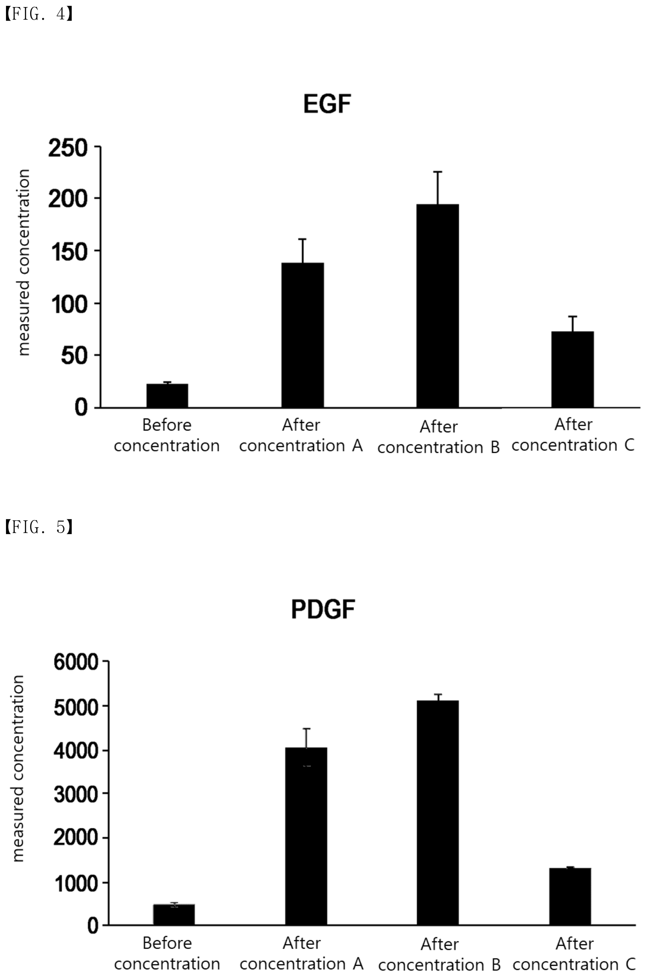

Using the aqueous two-phase system formed in the above Example 1, we concentrated regeneration factor-rich plasma (RRP). To confirm the concentration of the concentrated RRP, an EGF ELISA kit and a PDGF ELISA kit were used. As shown in Figures 4 and 5, although System B achieved approximately 8.7-fold concentration of EGF and approximately 10.518-fold concentration of PDGF compared to the pre-concentration levels, the most efficient concentration was achieved. However, System A, which formed an aqueous solution imbalance, showed approximately 5-fold concentration of EGF and approximately 8-fold concentration of PDGF compared to the pre-concentration levels, while System C also showed approximately 3-fold concentration of EGF and approximately 3-fold concentration of PDGF compared to the pre-concentration levels. Therefore, the aqueous two-phase system provided by the present invention has been confirmed to efficiently concentrate regeneration factor-rich plasma (RRP).

### [Example 31 Verification of the antioxidant effect of concentrated regeneration factor-rich plasma (Regeneration factor-rich Plasma, RRP)

To confirm the antioxidant effect of the concentrated regeneration factor-rich plasma (RRP) obtained in Example 2 on human skin cells, an MTT (3-(4,5-dimethylthiazol-2-yl)-2,5-diphenyltetrazolium bromide) assay was performed. In brief, HaCaT cells were cultured at a density of 5 ×10⁴ cells/mL in a 96-well plate for 24 hours. Subsequently, RRP at final concentrations of 0.1%, 1%, and 5% was treated with 1 mM H₂O₂ for 8 hours. A saline solution was used as a control. After RRP treatment, the medium was removed, and 4 µL of MTT reagent at 5 mg/mL was added, followed by incubation for 4 hours. After removing the medium, 100 µL of DMSO was added and dissolved for 10 minutes. The absorbance at a wavelength of 570 nm was measured to determine the cell survival rate.

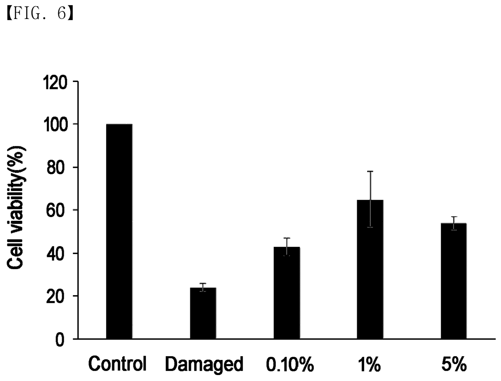

As shown in Figure 6, the RRP concentrated by Example 2 showed a survival rate that was at least 2-fold to over 3-fold higher than that of damaged cells at all concentrations (0.1%, 1%, and 5%). Thus, the RRP concentrated according to the aqueous two-phase system provided by the present invention is rich in regeneration factors, promotes cell proliferation, and is effective for regenerative therapy. Additionally, it exhibits antioxidant effects on human skin cells, confirming its usefulness for skin beauty.

Although the present invention has been described in detail above, the scope of the present invention is not limited thereto. It will be obvious to those skilled in the art that various modifications and changes can be made without departing from the technical spirit of the present invention described in the claims.

## Claims

1. A method for extracting or concentrating regeneration factor-rich plasma (RRP) from blood using an aqueous two-phase system.

2. The method according to claim 1,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

3. The method according to claim 2,
wherein the aqueous two-phase system is formed by adding at least one additive selected from the group consisting of sodium sulfate, polyethylene glycol (PEG), and hyaluronic acid.

4. The method according to claim 3,
wherein the sodium sulfate is present in a concentration of 1 to 10% (w/w).

5. The method according to claim 3,
wherein the polyethylene glycol (PEG) is present in a concentration of 5 to 20% (w/w).

6. The method according to claim 3,
wherein the hyaluronic acid is present in a concentration of 1 to 10% (w/w).

7. The method according to claim 6,
wherein the hyaluronic acid has an average molecular weight of 100,000 or more and 500,000 or less.

8. The method according to claim 7,
wherein the blood is plasma.

9. The method according to claim 8,
wherein the plasma is free of platelets or exosomes.

10. The method according to claim 9,
wherein the platelets or exosomes are removed by centrifugation or chromatography.

11. The method according to claim 10,
wherein the method for extracting or concentrating RRP further comprises a step of purifying impurities.

12. The method according to claim 11,
wherein the step of purifying the impurities comprises purifying the RRP using a chromatography filter immobilized with Apo A and Apo B antibodies.

13. The method according to claim 12,
wherein the extraction comprises selecting the RRP using a chromatography filter immobilized with anti-RRP antibodies.

14. A skin care method comprising regeneration factor-rich plasma (RRP) extracted or concentrated using the method according to any one of claims 1 to 13.

15. The method according to claim 14,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

16. A skin beauty cosmetic containing RRP (Regeneration factor-rich Plasma) extracted or concentrated using any method according to any one of claims 1 to 13.

17. The skin beauty cosmetic according to claim 16,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

18. A skin cosmetic composition comprising RRP (Regeneration Factor-rich Plasma) extracted or concentrated using the method according to any one of claims 1 to 13.

19. The skin cosmetic composition according to claim 18,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

20. A composition for treating a wound comprising regeneration factor-rich plasma (RRP) extracted or concentrated using the method according to any one of claims 1 to 13.

21. The composition for treating a wound according to claim 20,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

22. The composition for treating a wound according to claim 21,
wherein the wound is selected from the group consisting of a wound, a bedsore, a burn, an abrasion, a puncture ulcer, a stab wound, a chronic skin wound caused by reactive oxygen species, a contusion, a cut, a wound of the pharyngeal or oral mucosa, a laceration, a diabetic ulcer, a leg ulcer, a hypertensive ischemic ulcer, a venous ulcer, and a foot ulcer.

23. A composition for regeneration comprising regeneration factor-rich plasma (RRP) extracted or concentrated using the method according to any one of claims 1 to 13.

24. The composition for regeneration according to claim 23,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

25. The composition for regeneration according to claim 24,
wherein the regeneration is required for a condition selected from the group consisting of temporomandibular joint disorder, gastric ulcer caused by endoscopic submucosal dissection, human autologous fat grafting, treatment of knee osteoarthritis, treatment of chronic non-healing ulcers, hair regeneration therapy for androgenic alopecia, treatment of Achilles tendon rupture, high-risk proliferative diabetic retinopathy, proliferative diabetic retinopathy, knee osteoarthritis (OA), patellar dislocation, lumbar facet joint syndrome, retinitis pigmentosa, diabetic macular edema, traumatic bone cyst, proliferative diabetic retinopathy, scar, dry eye syndrome, Sjögren's syndrome, osteoarthritis, meniscal lesion, melasma, muscle injury, cubital tunnel syndrome, chronic ulcer, alopecia, epicondylitis, bone resorption, rotator cuff tear, thin endometrium, striae gravidarum, plantar fasciitis, coxarthrosis, osteoarthritis, muscle rupture, tennis elbow, skin grafting, aging, knee arthritis, burn, renal failure, and atopic disease.

26. A vessel for extracting or concentrating RRP,
wherein the vessel used for extracting or concentrating regeneration factor-rich plasma (RRP) according to any one of claims 1 to 13, comprising:
a body providing an internal space for receiving blood and extracting or concentrating the RRP upon centrifugation;
a first lead provided on one side of the body for injecting the blood or another substance into the internal space of the body; and
a second lead provided on the other side of the body, opposite the one side, for discharging the RRP extracted or concentrated from the body.

27. A vessel according to claim 26,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

28. The vessel according to claim 27,
wherein the first lid is provided on a cover provided on the upper side of the body to open and close the body.

29. The vessel according to claim 28,
wherein the other side of the body is formed to narrow toward the second lead.

30. The vessel according to claim 29,
wherein the vessel further comprising a support that surrounds the other outer surface of the body around the second lead.

31. A wound treatment method comprising regeneration factor-rich plasma (RRP) extracted or concentrated using the method according to any one of claims 1 to 13.

32. A skin regeneration method comprising regeneration factor-rich plasma (RRP) extracted or concentrated using the method according to any one of claims 1 to 32.

33. An anti-aging cosmetic composition comprising regeneration factor-rich plasma (RRP) extracted or concentrated using the method according to any one of claims 1 to 32.

34. The anti-aging cosmetic composition according to claim 33,
wherein the regenerative factor is at least one regenerative factor selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

35. An anti-aging treatment method comprising administering to a subject regeneration factor-rich plasma (RRP) extracted or concentrated using the method according to any one of claims 1 to 13.
